# EUROPEAN PATENT APPLICATION

(11) **EP 3 767 637 A1**
(43) Date of publication of application: **20.01.2021**
(21) Application number: 20185305.8
(22) Date of filing: 10.07.2020
(51) Int. Cl.: G16H 50/30, A61B 5/00

(54) **PERSONALIZED BASELINES, VISUALIZATIONS, AND HANDOFFS**

(30) Priority: 15.07.2019 US 201962874059 P; 26.03.2020 US 202062994917 P
(71) Applicant: Hill-Rom Services, Inc., Batesville, IN 47006-9167 (US)
(72) Inventor: FAYETTEVILLE, Jotpreet Chahal, Batesville, IN 47006-9167 (US); SYRACUSE, Kathryn M. Coles, Batesville, IN 47006-9167 (US); BATESVILLE, Kirsten M. Emmons, Batesville, IN 47006-9167 (US); DEWITT, Stacey A. Fitzgibbons, Batesville, IN 47006-9167 (US); SYRACUSE, Craig M. Meyerson, Batesville, IN 47006-9167 (US); MILROY, Lori Ann Zapfe, Batesville, IN 47006-9167 (US)
(74) Representative: Findlay, Alice Rosemary

(57) **Abstract**

A system for determining personalized baselines for use across multiple locations and by multiple caregivers. The system aggregates physiological parameters measured at a first location, determines a personalized baseline based on the aggregated physiological parameters, and generates a user interface that compares physiological parameters measured at a second location to the personalized baseline.

## Description

It can be challenging to determine whether a patient's vital signs are higher or lower than what is normal for that patient. For example, a systolic blood pressure of 100 mmHg may be low for a patient who typically has a systolic blood pressure of 120 mmHg while a systolic blood pressure of 100 mmHg may be normal for another patient.

Measuring vital signs without knowing a patient's baseline vital signs make it difficult for caregivers to provide care and act for the patient. For example, without knowing whether the vital signs of a patient are above or below their baseline or average, it is difficult for a caregiver to determine whether to initiate a treatment or when to stop a treatment.

Further complications arise from the fact that many systems are not interoperable such that vital signs obtained in one location such as the office of the patient's primary care physician are not transferable to another location such as the hospital where the patient is scheduled to have surgery. This problem exists not only for vital sign baseline data but for other patient contextual data that may be important for treating the patient.

The lack of data consolidation and availability may cause relevant data to be missed when evaluating a patient, during patient handoff, or during a patient transfer. This may cause delays in detecting when a patient's condition is deteriorating.

In one aspect, a system is disclosed for determining personalized baselines for use across multiple locations and by multiple caregivers. The system comprises at least one processor; and memory encoding instructions which, when executed by the at least one processor, cause the at least one processor to aggregate physiological parameters measured at a first location; determine a personalized baseline based on the aggregated physiological parameters; and generate a user interface that compares physiological parameters measured at a second location to the personalized baseline.

In certain embodiments, the system further comprises at least one sensor configured to collect at least one of the physiological parameters continuously or periodically.

In certain embodiments, the system further comprises a server on which the personalized baselines are determined.

In certain embodiments, the personalized baseline is at least one of: an average value; a range of values; a median value; or a most frequently repeated value, of the aggregate physiological parameters.

In certain embodiments, the first location is different from the second location, and the first and second locations are selected from a group consisting of a hospital, long-term-care facility, skilled nursing facility, physician office, and a patient's home.

In certain embodiments, the personalized baselines are customizable based on a status of a patient including when the patient is at rest, when the patient is active, after the patient concludes an activity, and when the patient is sleeping. The personalized baselines are customizable based on previous hospitalization discharge data. The system analyzes changes to the personalized baselines by one or more types of interventions.

In certain embodiments, the system assigns confidence intervals to the physiological parameters measured at the first location, the confidence intervals assigned based on the type of device used to measure the physiological parameters. The user interface can include one or more windows that display visualization curves that compare the physiological parameters measured at the second location to the personalized baselines. The personalized baselines may be displayed in the one or more windows as boundaries representing the confidence intervals. In certain examples, the personalized baseline is an average value. In certain examples, the personalized baseline is a range of values.

In certain embodiments, personalized baselines are generated for physiological parameters including heart rate, respiration rate, systolic and diastolic blood pressure, temperature, blood oxygen saturation (SpO2), end-tidal CO2 (EtCO2), electrocardiogram (ECG) data, blood glucose level, partial thromboplastin time (PTT), prothrombin time (INR), hemoglobin level (A1c), patient weight, and patient movement.

In certain embodiments, the system further comprises bed motion sensors to detect patient movement data including bed egress and ingress, duration of time spent in bed, and the positioning of the bed. The physiological parameters include the patient movement data, and the patient movement data is used to generate a daily mobility pattern as a personalized baseline for the patient. The mobility of the patient during a 24 hour period can be compared to the daily mobility pattern to determine a likelihood of patient deterioration. The comparison can be displayed in the user interface, and the user interface can be generated on a device in the first or second location.

In another aspect, a method of comparing a measured physiological parameter to a personalized baseline of a patient is disclosed. The method comprises aggregating physiological parameter data over a period of time at one or more first locations, determining a personalized baseline based on the aggregated physiological parameter data, and generating a user interface that displays a comparison of a physiological parameter measured at a second location to the personalized baseline. In certain examples, the first location is different from the second location, and the first and second locations are a hospital, a long-term-care facility, a skilled nursing facility, a physician's office, or the patient's home.

In certain embodiments, the method can further comprise displaying a visualization curve comparing the physiological parameter measured at the second location to the personalized baseline in a window of the user interface.

In certain embodiments, the physiological parameter data includes patient movement data, and the method further comprises using the patient movement data to generate a daily mobility pattern as a personalized baseline for the patient. The method can further comprise comparing mobility of the patient during a 24 hour period to the daily mobility pattern to determine a likelihood of patient deterioration. The method can further comprise displaying the comparison of the mobility during the 24 hour period to the daily mobility pattern in the user interface, the user interface being generated on a device in the first or second location.

In certain embodiments, the method can further comprise determining personalized baselines for physiological parameters including heart rate, respiration rate, blood pressure, temperature, blood oxygen saturation (SpO2), end-tidal CO2 (EtCO2), electrocardiogram (ECG) data, blood glucose level, partial thromboplastin time (PTT), prothrombin time (INR), hemoglobin level (A1c), patient weight, and patient movement.

In certain embodiments, the method further comprises assigning confidence intervals to the physiological parameter data depending on the type of device used to measure the physiological parameter data. In certain embodiments, the method can further comprise customizing the personalized baselines based on a status of a patient including when the patient is at rest, when the patient is active, after the patient concludes an activity, and when the patient is sleeping. In certain embodiments, the method can further comprise customizing the personalized baselines based on previous hospitalization discharge data.

The invention will now be further described by way of example with reference to the accompanying drawings, in which:
FIG. 1 is a schematic diagram of a system for determining personalized baselines for use across multiple care locations and by multiple caregivers.
FIG. 2 is an example user interface that includes one or more windows for displaying measured vital signs next to corresponding personalized baselines.
FIG. 3 illustrates a method of comparing a measured vital sign to a personalized baseline of a patient.
FIG. 4 illustrates an example user interface that displays visualization curves comparing physiological parameters to personalized baselines.
FIG. 5 illustrates another example user interface that displays visualization curves comparing physiological parameters to personalized baselines.
FIG. 6 illustrates an example handoff user interface.
FIG. 7 illustrates example physical components of a computing device.
FIG. 8 schematically illustrates a diagram of behavioral changes by a patient that can influence the patient's daily routine and patterns of daily living.
FIG. 9 illustrates a table that compares the onset of acute illnesses, clinical symptoms, and potential shifts in bed mobility patterns.
FIG. 10 illustrates a chart comparing a daily mobility pattern with the mobility of a patient during various 24 hour periods of time.
FIG. 11 illustrates a chart that displays a 30 day average number of movements per hour in bed during the day, evening, and night.
FIG. 12 illustrates a chart that displays a 7 day average number of movements per hour in bed during the day, evening, and night.
FIG. 13 illustrates a chart that displays an average number of movements per hour in bed during the day, evening, and night from a previous day.
FIG. 14 illustrates a chart that displays an average number of movements per hour in bed during the day, evening, and night from a current day.

Various embodiments and advantages are explained more fully with reference to the non-limiting examples that are described and illustrated in the accompanying drawings and detailed in the following description. The features illustrated in the drawings are not necessarily drawn to scale, and features of one embodiment may be employed with other embodiments, even if not explicitly stated herein.

The examples used herein are intended merely to facilitate an understanding of ways in which the claimed subject matter may be practiced and to enable those of skill in the art to practice the embodiments of the claimed subject matter described herein. The embodiments provided herein are merely illustrative and should not be construed as limiting the scope of the claimed subject matter, which is defined solely by the appended claims. Also, like reference numerals may represent similar parts throughout the several views of the drawings.

The present disclosure describes improved systems and methods for determining personalized baselines for patients who have chronic diseases or have a known scheduled surgery. The personalized baselines can be shared across multiple locations for use by multiple caregivers to assist the caregivers in detecting when a patient's condition is deteriorating and thus help reduce negative patient outcomes. The systems and methods allow for different patients to be monitored with varying levels of scrutiny, based at least in part on the personalized baselines and the needs of the individual patients, and facilitate efficient and effective monitoring of multiple patients across various care locations.

In the examples provided herein, the systems and methods utilize data from disparate sources and process that data efficiently to determine the personalized baselines. In these examples, physiological data from one or more devices and sensors is processed, and the personalizes baselines are calculated for a patient. The personalized baselines are used in practical applications, including the generation of one or more user interfaces which can be used by caregivers to provide patient care across various care locations.

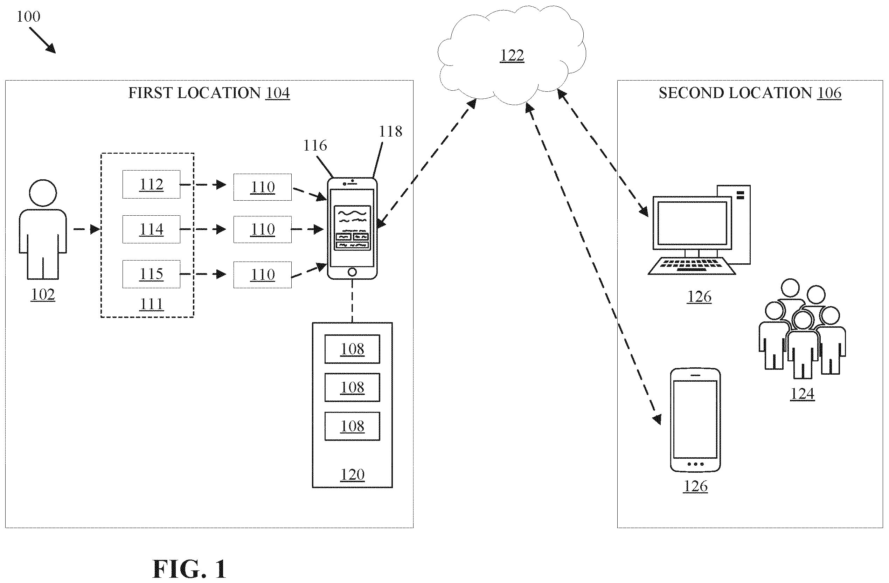

FIG. 1 is a schematic diagram of a system 100 that determines personalized baselines for use across multiple care locations and by multiple caregivers. The personalized baselines are patient-centric such that the personalized baselines are unique to a particular patient. The personalized baselines are generated for one or more physiological parameters.

The system 100 generates personalized baselines for a patient 102 in a first location 104 that can be used by caregivers 124 in a second location 106. In one example, the first location 104 is the home of the patient 102. In another example, the first location 104 is the office of the primary care physician of the patient 102. In a further example, the second location 106 is a hospital where the patient 102 is scheduled to have surgery. While only a single first location 104 is illustrated in the example embodiment depicted in FIG. 1, it is contemplated that the personalized baselines determined by the system 100 can be obtained from a plurality of first locations 104. Similarly, while only a single second location 106 is illustrated in the example embodiment depicted in FIG. 1, it is contemplated that the personalized baselines determined by the system 100 can be used by a plurality of caregivers in multiple second locations 106 outside of one or more first locations 104.

The system 100 uses one or more devices 110 in the first location 104 to collect continuous or periodical physiological parameters 111 for a given period of time, that is, physiological parameters 111 may be collected continuously, i.e. without interruption, or periodically, i.e. intermittently. As shown in FIG. 1, the physiological parameters 111 include vital signs data 112, patient movement data 114, and additional parameters 115. As an illustrative example, the system 100 obtains vital signs data 112 over a period of six months during primary care visits or from one or more devices 110 in the home of the patient 102. The vital signs data 112 obtained from the devices 110 includes any one or more of the following: heart rate data, respiration rate data, temperature data, pulse oximetry data, blood pressure data (including systolic and diastolic blood pressure), blood oxygen saturation (SpO2) data, end-tidal CO2 (EtCO2) data, electrocardiogram (ECG) data, and the like.

In some embodiments, the one or more devices 110 also obtain patient movement data 114 for a given period of time. As an illustrative example, the one or more devices 110 can include one or more types of movement tracking devices including GPS tracking devices, one or more accelerometers, or other motion-detecting technologies.

In some embodiments, the additional parameters 115 include lab results such as blood glucose level, partial thromboplastin time (PTT), prothrombin time (INR), and hemoglobin levels (A1c) obtained from blood analyses performed by the one or more devices 110. In some embodiments, the additional parameters 115 include patient weight. As described above, the vital signs data 112, patient movement data 114, and additional parameters 115 are physiological parameters that are used to determine one or more personalized baselines for a particular patient.

In some embodiments, the devices 110 include a specialized vital signs patch (VSP) that is wearable by the patient 102 to obtain the vital signs data 112. In some embodiments, the one or more devices 110 include consumer grade devices such as wearable devices that incorporate fitness tracking and health-oriented capabilities including wearable activity trackers and smartwatches. In some embodiments, the one or more devices 110 are medical grade devices approved by the Food and Drug Administration (FDA). In further embodiments, the one or more devices 110 include ambulatory electrocardiography devices such as a Holter monitor for cardiac monitoring for a given period of 24 to 48 hours.

In some embodiments, a confidence interval is assigned to the vital signs data 112 obtained from the one or more devices 110 depending on the type of device. For example, higher confidence intervals are assigned to data obtained from medical grade devices whereas lower confidence intervals are assigned to data obtained consumer grade devices.

Still referring to FIG. 1, the physiological parameters 111 obtained from the one or more devices 110 is accumulated by an electronic device 116. In the example embodiment illustrated in FIG. 1, the electronic device 116 is smartphone. In other embodiments, additional types of electronic devices may be used to accumulate the physiological parameters 111. The connections between the one or more devices 110 and the electronic device 116 include wireless connections such as WiFi®, Bluetooth®, cellular networks, the Internet, and the like. In some embodiments, the one or more devices 110 are embedded in the electronic device 116 such that the electronic device 116 collects the vital signs data 112 and patient movement data 114 from the patient 102 directly without requiring a wireless connection.

The electronic device 116 includes a computing device having at least one processor and a memory. Stored in the memory of the electronic device 116 is an application 118 connected to a cloud server 122 that generates the personalized baselines 108. The application 118 utilizes an algorithm that aggregates the physiological parameters 111 to generate one or more personalized baselines 108 for one or more physiological parameters of the patient 102. For example, the application 118 can generate a personalized baseline 108 for the heart rate, respiration rate, and systolic and diastolic blood pressures of the patient 102. Additional personalized baselines for additional vital signs are contemplated.

As an illustrative example, the application 118 can aggregate systolic blood pressure data over a six month period during visits to the office of the patient's primary care physician and/or from consumer blood pressure monitoring devices at the home of the patient. The algorithm may assign confidence intervals to the systolic blood pressure data depending on the type of device used to measure the data. The algorithm calculates an average value, standard deviation, minimum value, maximum value, median value, and mode value (i.e., the value that appears most often) from the systolic blood pressure data of the patient 102, and determines a personalized baseline 108 for the systolic blood pressure of the patient 102 based on one or more of these calculations. While the foregoing example is described with respect to a personalized baseline 108 for systolic blood pressure, personalized baselines for other vital signs and physiological parameters can be determined in a similar manner.

In addition to the illustrative example described above, the application 118 also generates personalized baselines 108 based on the patient movement data 114 and additional parameters 115. As an illustrative example, the application 118 generates a personalized baseline 108 for the number of steps that the patient 102 walks during a specified period of time (e.g., 24 hours) based on the patient movement data 114. As another example, the application 118 generates personalized baselines 108 for the blood glucose level, partial thromboplastin time (PTT), prothrombin time (INR), and hemoglobin levels (A1c).

In some embodiments, the application 118 calculates the personalized baseline 108 as an average value. In some embodiments, the application 118 calculates the personalized baseline 108 as a range of values. In further embodiments, the application 118 calculates the personalized baseline 108 as a median value or a most frequently repeated value.

In some embodiments, in addition to aggregating the physiological parameters 111 from the devices 110, the application 118 considers prior hospitalizations of the patient 102 and the physiological parameters taken from the patient 102 before being discharged from prior hospitalizations.

In some embodiments, the application 118 considers physiological parameters from patients of similar age, body mass index (BMI), medical condition, gender, and the like to generate population type estimated baselines instead of, or in addition to, the personalized baseline 108 of the patient 102.

The personalized baselines 108 generated by the application 118 are customizable based on the status of the patient 102 including when the patient 102 is at rest, when the patient 102 is active, after the patient 102 concludes an activity, and when the patient 102 is sleeping. In some example embodiments, the personalized baselines are customizable based on previous hospitalization discharge data. Additional customizations based on the status of the patient are contemplated.

The application 118 determines the impact of one or more types of interventions on the personalized baselines 108. As an illustrative example, an intervention may include taking a medication, and the application 118 monitors the personalized baselines 108 of the patient 102 before and after the medication is taken. Additional types of interventions are contemplated.

The application 118 generates a report 120 that includes the personalized baselines 108 of the patient 102, and the electronic device 116 acts as a gateway to transfer the report 120 to the cloud server 122. The electronic device 116 may use various telecommunications networks to transfer the report 120 to the cloud server 122 including cellular networks and the internet. In alternative embodiments, the cloud server 122 receives the physiological parameters 111 from the electronic device 116, and the cloud server 122 generates the personalized baselines 108 and the report 120 using the physiological parameters 111. In some embodiments, the cloud server 122 further analyzes the personalized baselines 108 to determine trends and risk profiles for the patient 102.

As shown in FIG. 1, the second location 106 includes one or more terminals 126 that have access to the cloud server 122 via the internet and/or cellular networks. The one or more terminals 126 include desktop computers, tablet computers, smartphones, and the like.

Multiple caregivers 124 at the second location 106 may utilize the one or more terminals 126 to view the report 120 that includes the personalized baselines 108 of the patient 102. In some embodiments, the report 120 is viewed on the application 118 which is accessible via the one or more terminals 126. In some embodiments, the report 120 is viewed via email received on the one or more terminals 126 in the second location 106.

The trend analysis and risk profile determinations performed by the cloud server 122 are also displayed on the one or more terminals 126 in the second location 106 for the caregivers 124 to take one or more actions. For example, when it is determined that the patient 102 in the first location 104 is at risk for deterioration, the caregivers 124 may take immediate action such as calling the patient 102 directly, dispatching a homecare nurse or EMT to treat the patient 102, or sending notifications to the mobile phone of the patient 102.

In one illustrative example, the cloud server 122 determines that the patient 102 is at risk for congestive heart failure based on accumulated patient specific data that has been gathered from the patient's electronic medical record (EMR). The cloud server 122 trends the patient's weight from a device 110 in the patient's home on a daily basis and triggers an alert to the caregivers 124 (or a remote patient monitoring company) when the patient 102 gains weight beyond a threshold limit within a period of time such as when the patient 102 gains 3 lbs. or more in 24 hours, or when the patient 102 gains 5 lbs. or more in one week.

In another illustrative example, a device 110 in the patient's home measures the blood glucose level of the patient 102 on a daily basis, and the blood glucose level measurements are sent to the cloud server 122. Thereafter, the cloud server 122 determines that blood glucose level of the patient 102 is too low by comparing the blood glucose level measurements to a personalized baseline 108 for the patient 102. The cloud server 122 sends a message to the patient 102 that includes instructions such as to drink juice and eat protein, and thereafter assesses the patient's blood glucose level in 30 minutes. The cloud server 122 may also send a secondary message to the patient's homecare nurse so that the homecare nurse prioritizes patient visits based on the status of the patient 102.

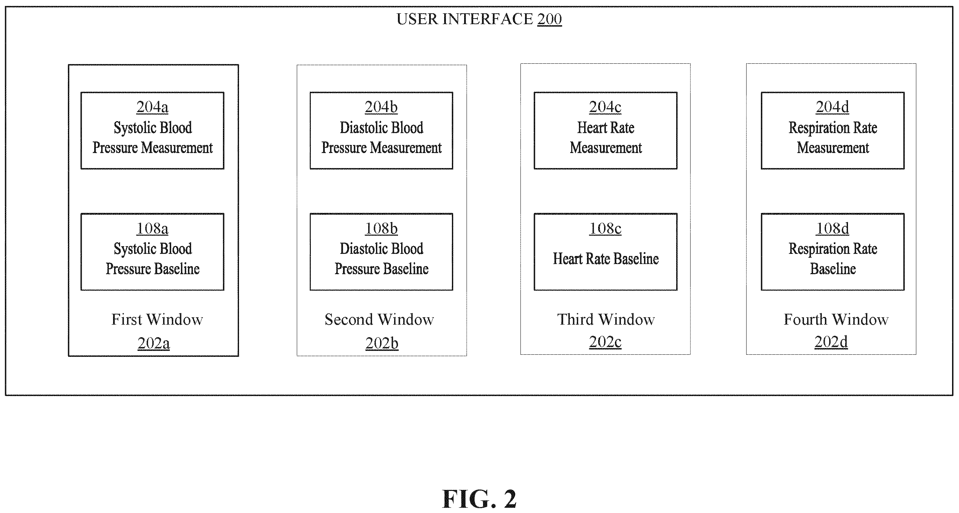

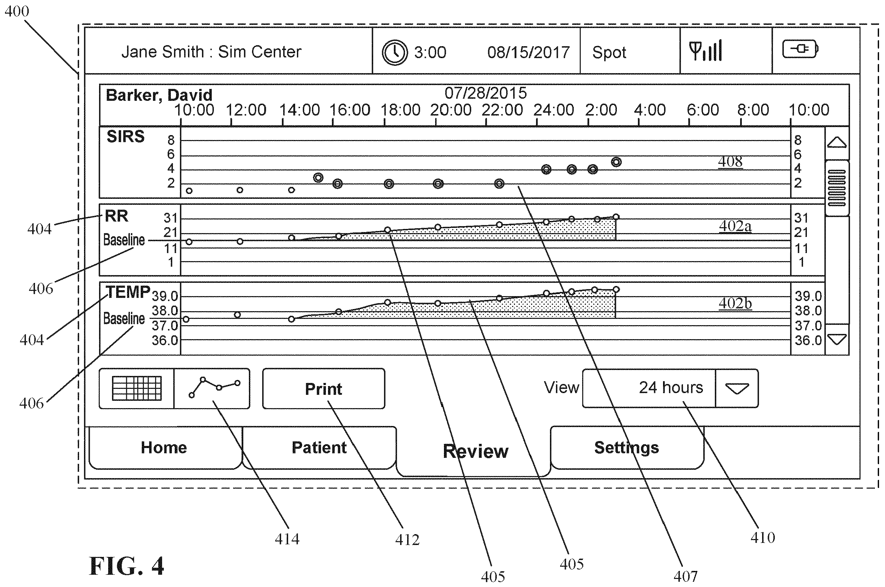

FIG. 2 is an example user interface 200 that includes one or more windows 202 that display measured physiological parameters 204 obtained at the second location 106 next to the personalized baselines 108 of the patient 102. The user interface 200 is generated by the application 118. An additional user interface 400 is described with reference to FIG. 4.

The user interface 200 enables caregivers to compare the measured physiological parameters 204 to the personalized baselines 108 to help the caregivers decide whether to start a treatment or end a treatment based on the comparison. For example, when a comparison shows that a measured physiological parameters 204 is higher or lower than a corresponding personalized baseline 108, the caregiver can decide to take an action to prevent patient deterioration.

In the example illustrated in FIG. 2, the user interface 200 includes a first window 202a that displays a measured systolic blood pressure 204a next to a systolic blood pressure baseline 108a, a second window 202b that displays a measured diastolic blood pressure 204b next to a diastolic blood pressure baseline 108b, a third window 202c that displays a measured heart rate 204c next to a heart rate baseline 108c, and a fourth window 202d that displays a measured respiration rate 204d next to a respiration rate baseline 108d. It is contemplated that the user interface 200 is configurable to display additional windows that compare additional types of measured physiological parameters and corresponding personalized baselines, and is also configurable to display fewer windows. Also, the placement of the measured vital signs with respect to the corresponding personalized baselines in the windows may vary such that the measured physiological parameters may be displayed above, below, to the right, to the left etc. of the personalized baselines.

In some embodiments, the user interface 200 displays the personalized baselines 108a-108d as an average value. In some embodiments, the user interface 200 displays the personalized baselines 108a-108d as a range of values. In some embodiments, the user interface 200 further displays a standard deviation, minimum value, maximum value, and/or a histogram of the most frequently repeated values for the personalized baselines 108a-108d. In further embodiments, the user interface 200 displays within each window 202 a visualization curve that compares measured physiological parameters and the personalized baselines for the patient 102. Such visualization curves are illustrated the user interface 400 of FIG. 4, which is described in greater detail below.

The user interface 200 displays the appropriate personalized baselines depending on the condition and/or status of the patient 102 described above. As an illustrative example, when the patient 102 has been resting in bed for a while, the user interface 200 displays the personalized baselines 108a-108d as calculated when the patient 102 was at rest. As a further illustrative example, when the patient 102 is sleeping, the user interface 200 displays the personalized baselines 108a-108d as calculated when the patient 102 was sleeping.

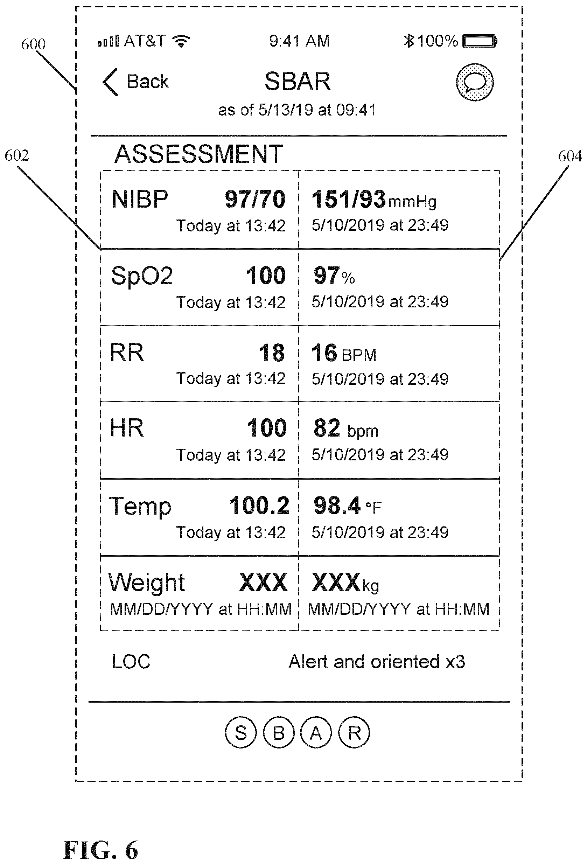

In some embodiments, the user interface 200 is part of a handoff screen that is initiated by a caregiver when the caregiver begins their shift. The handoff screen pulls relevant patient data including the personalized baselines 108a-108d and displays it. An example handoff screen is described in greater detail below with reference to FIG. 6.

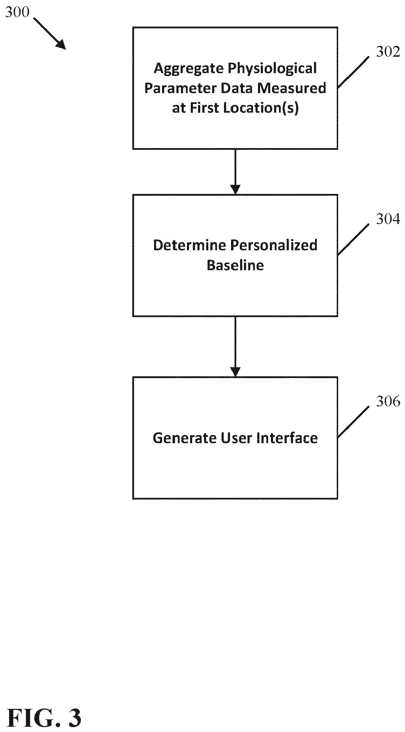

FIG. 3 illustrates a method 300 of comparing a measured physiological parameter to a personalized baseline. The method 300 includes aggregating physiological parameter data over a period of time at one or more first locations (Step 302), determining a personalized baseline based on the aggregated physiological parameter data (Step 304), and generating a user interface that displays a comparison of a physiological parameter measured at a second location to the personalized baseline (Step 306).

At Step 302, the physiological parameter data is aggregated from one or more devices at one or more first locations. First locations may include the home of a patient and/or the office of the patient's primary care physician. During Step 302, the physiological parameter data can be aggregated from consumer medical devices and medical grade devices. In some embodiments, Step 302 includes assigning confidence intervals to the physiological parameter data depending on the type of device used to measure the physiological parameter data at the one or more first locations. For example, higher confidence intervals are assigned to data obtained from medical grade devices whereas lower confidence intervals are assigned to data obtained consumer grade devices. During Step 302, the physiological parameter data is aggregated over a period of time such as one or more weeks or months.

At Step 304, personalized baselines for one or more physiological parameters are determined based on the aggregated physiological parameter data. Step 304 can include determining personalized baselines for heart rate, respiration rate, systolic and diastolic blood pressures, temperature, blood oxygen saturation (SpO2), end-tidal CO2 (EtCO2), electrocardiogram (ECG) data, blood glucose level, partial thromboplastin time (PTT), prothrombin time (INR), hemoglobin level (A1c), patient weight, and patient movement data. An algorithm uses the confidence intervals to weigh the physiological parameter data, and calculate an average value, standard deviation, minimum value, maximum value, and median value for the vital signs data. In some embodiments, Step 304 includes calculating the personalized baselines as an average value based on the aggregated physiological parameter data. In some embodiments, Step 304 includes calculating the personalized baselines as a range of values based on the aggregated physiological parameter data.

At step 306, a user interface is generated for displaying a comparison of a physiological parameter to a personalized baseline. The user interface may include one or more windows for displaying a comparison of the physiological parameter measured at the second location and the personalized baseline. The user interface is viewable by one or more caregivers at the second location, enabling the caregivers to take one or more actions based on the comparison such as calling the patient directly, dispatching a homecare nurse or EMT to treat the patient, or sending notifications to the mobile phone of the patient.

FIG. 4 illustrates an example user interface 400 that displays visualization curves comparing physiological parameters to personalized baselines. As shown in the example illustrated in FIG. 4, a first window 402a includes a visualization curve 405 that compares a first physiological parameter such as respiration rate to a personalized baseline 406, and a second window 402b includes a visualization curve 405 that compares a second physiological parameter such as temperature to a personalized baseline 406.

In the example illustrated in FIG. 4, the user interface 400 includes a window 408 that displays a visualization curve 407 for an early warning score 409. In the illustrated example, a visualization curve 407 for a systemic inflammatory response syndrome (SIRS) score is displayed in the window 408. A visualization curve 407 for additional types of early warning scores 409 can be displayed in the window 408 including, for example, modified early warning scores (MEWS), national early warning scores (NEWS), modified early obstetric warning scores (MEOWS), pediatric early warning scores (PEWS), and the like.

The visualization curves 405, 407 are displayed above and below one another to provide an intuitive visual comparison of the physiological parameters 404, personalized baselines 406, and early warning score 409, to provide further context and information regarding the status and condition of a patient that is being monitored by a caregiver.

In some examples, the user interface 400 includes a selectable icon 410 that when selected can be used to adjust the period of time for the visualization curves 405, 407 that are displayed in the windows 402a, 402b, and 408. For example, the visualization curves 405, 407 can be displayed for a period longer than 24 hours or a period shorter than 24 hours.

In some examples, the user interface 400 includes a selectable icon 412 that when selected can be used to print the visualization curves 405, 407. In some examples, the user interface 400 includes a selectable icon 414 that when selected can be used to adjust the type of visualization curve 405, 407 displayed in the windows 402a, 402b, and 408.

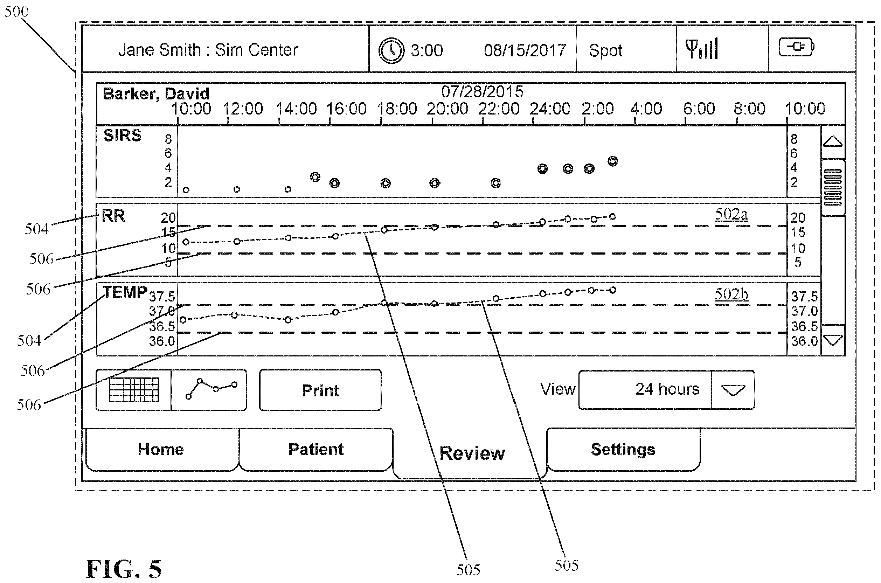

FIG. 5 illustrates another example user interface 500 that displays visualization curves comparing physiological parameters to personalized baselines. The user interface 500 shares many features with the user interface 400 described above. As shown in the example illustrated in FIG.5, a first window 502a includes a visualization curve 505 that displays a first physiological parameter 504 such as respiration rate within boundaries 506, and a second window 502b includes a visualization curve 505 that displays a second physiological parameter such as temperature within boundaries 506. The boundaries 506 define a swim lane representing a confidence interval for the personalized baselines determined for the respiration rate and temperature, respectively, within the windows 502a, 502b.

FIG. 6 illustrates an example of a handoff user interface 600. The handoff user interface 600 can be part of a series of screens that form a situation, background, assessment, and recommendation (SBAR) user interface flow. In the example illustrated in FIG. 6, the handoff user interface 600 is an assessment screen where a first column of physiological parameters 602 are displayed next to a second column of physiological parameters 604.

The first column of physiological parameters 602 include current physiological parameters. The second column of physiological parameters 604 includes personalized baselines or previously measured physiological parameters. In the example illustrated in FIG. 6, the first column of physiological parameters 602 includes the date and time to indicate when each physiological parameter was last updated. The second column of physiological parameters 604 also includes the date and time establishing when the personalized baselines or previously measured physiological parameters were last determined or updated.

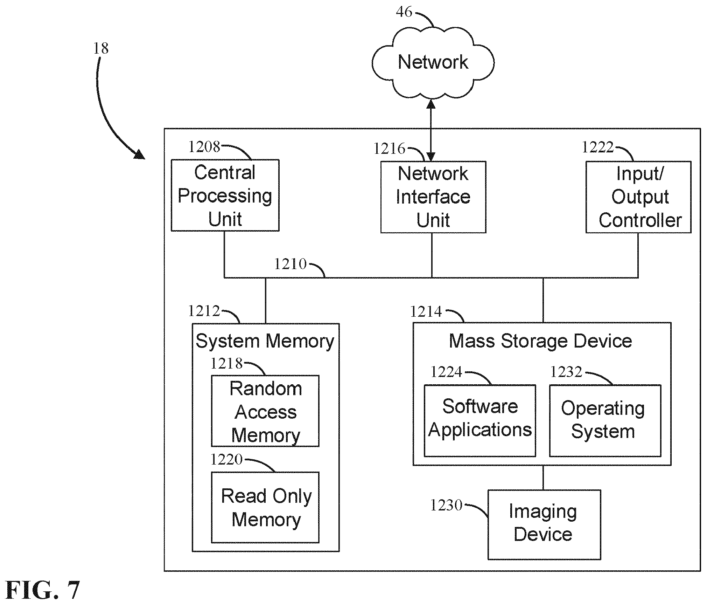

FIG. 7 illustrates example physical components of a computing device associated with the devices described above, including the electronic device 116 and terminals 126. As illustrated, the computing device includes at least one processor or central processing unit ("CPU") 1208, a system memory 1212, and a system bus 1210 that couples the system memory 1212 to the CPU 1208. The system memory 1212 includes a random access memory ("RAM") 1218 and a read-only memory ("ROM") 1220. A basic input/output system containing the basic routines that help to transfer information between elements within the computing device, such as during startup, is stored in the ROM 1220. The computing device further includes a mass storage device 1214 able to store software instructions and data. The central processing unit 1208 is an example of a processing device.

The mass storage device 1214 is connected to the CPU 1208 through a mass storage controller (not shown) connected to the system bus 1210. The mass storage device 1214 and its associated computer-readable data storage media provide non-volatile, non-transitory storage for the computing device. Although the description of computer-readable data storage media contained herein refers to a mass storage device, such as a hard disk or CD-ROM drive, it should be appreciated by those skilled in the art that computer-readable data storage media can be any available non-transitory, physical device or article of manufacture from which the device can read data and/or instructions. The mass storage device 1214 is an example of a computer-readable storage device.

Computer-readable data storage media include volatile and non-volatile, removable and non-removable media implemented in any method or technology for storage of information such as computer-readable software instructions, data structures, program modules or other data. Example types of computer-readable data storage media include, but are not limited to, RAM, ROM, EPROM, EEPROM, flash memory or other solid-state memory technology, CD-ROMs, digital versatile discs ("DVDs"), other optical storage media, magnetic cassettes, magnetic tape, magnetic disk storage or other magnetic storage devices, or any other medium which can be used to store the desired information and which can be accessed by the computing device.

According to various embodiments, the computing device may operate in a networked environment using logical connections to remote network devices through the network 46, such as a local network, the Internet, or another type of network. The computing device connects to the network 46 through a network interface unit 1216 connected to the system bus 1210. The network interface unit 1216 may also be utilized to connect to other types of networks and remote computing systems. The computing device also includes an input/output controller 1222 for receiving and processing input from a number of other devices, including a camera, a keyboard, a mouse, a touch user interface display screen, or another type of input device. Similarly, the input/output controller 1222 may provide output to a touch user interface display screen, a printer, or other type of output device.

The computing device may also include an optional imaging device 1230, such as a camera that is configured to capture still or moving images (i.e., video). The camera can be configured to capture high resolution images or video (e.g., 100-200+ fps) that can be used to conduct one or more of the analyses described herein.

As mentioned above, the mass storage device 1214 and the RAM 1218 of the device can store software instructions and data. The software instructions include an operating system 1232 suitable for controlling the operation of the device. The mass storage device 1214 and/or the RAM 1218 also store software instructions, that when executed by the CPU 1208, cause the computing device to provide the functionality discussed in this document.

In another aspect, the present disclosure relates to early prediction of acute deterioration in environments such as long term care facilities, skilled nursing facilities, long-term acute care hospitals (LTACH), and home care environments. In these environments, care is provided to an increasingly complex and aged population due to advances in medicine and changes in reimbursement structures. However, these types of environments typically have limited tools to identify early patient deterioration in a vulnerable patient population.

The detection of clinical deterioration in long-term care environments can be more complicated than in an acute care setting such as in a hospital at least because the patients in these types of environments are often cognitively impaired and unable to interpret and report changes in their health, and may also have impaired communication abilities. Additionally, clinical assessments in long term care environments are far less frequent and thorough then in an acute facility because long term care environments often experience high patient-caregiver ratios, constant caregiver/staff turnover, lower training standards, and fewer clinical tools. Additionally, patients in a home care setting may have only once a week interaction with a licensed nurse, and there may be no continuity among other assigned caregivers.

In this embodiment, long term personalized in-bed mobility patterns and ingress/ egress patterns are used to identify potential deterioration of patients in environments such as long term care facilities, skilled nursing facilities, and home care environments. Patients in these environments are generally debilitated individuals that follow a daily routine that is bed centric. For example, patients in these environments typically spend about 11-17 hours per day in bed. An acute illness will typically cause changes in their daily routine and patterns of daily living such as morning wake up times, personal hygiene routines, bathroom intervals, mealtimes, naps, bedtimes, recreational activities, and the like. The time, duration, and frequency of these activities may become altered when a patient becomes acutely ill.

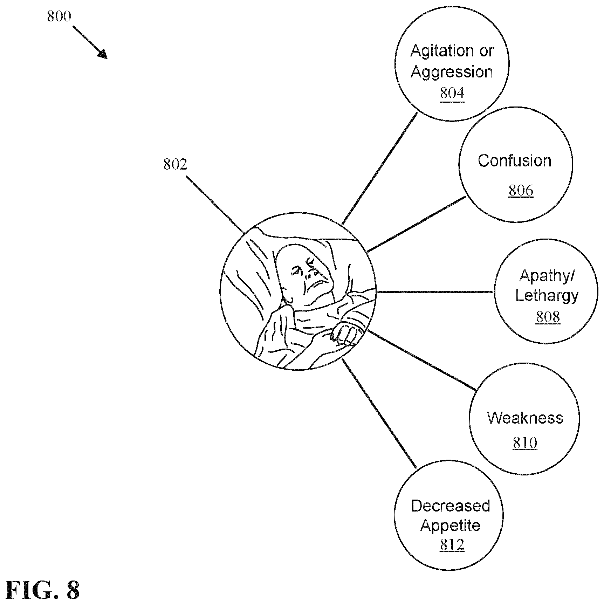

FIG. 8 schematically illustrates a diagram 800 of behavioral changes by a patient 802 that can influence the patient's daily routine and patterns of living. Behavioral changes experienced by the patient 802 can include, without limitation, agitation or aggression 804, confusion 806, lethargy 808, weakness 810, and decreased appetite 812. A patient who develops an acute illness while admitted in a facility such as long term care facility is more likely to exhibit behavioral changes than a patient who does not develop an acute illness.

Behavioral changes experienced by the patient 802 that can lead to changes in daily routines and patterns of living can include, without limitation, more non-purposeful movement and more transitions such as from increased agitation or aggression 804, daily pattern shifts and random or unusual motion from confusion 806, less self-initiated motion and hypoactive compared to baseline such as from lethargy 808, increased time spent in bed, increased time between transitions, less self-initiated motion from weakness 810, in bed during meals from decreased appetite 812, and the like.

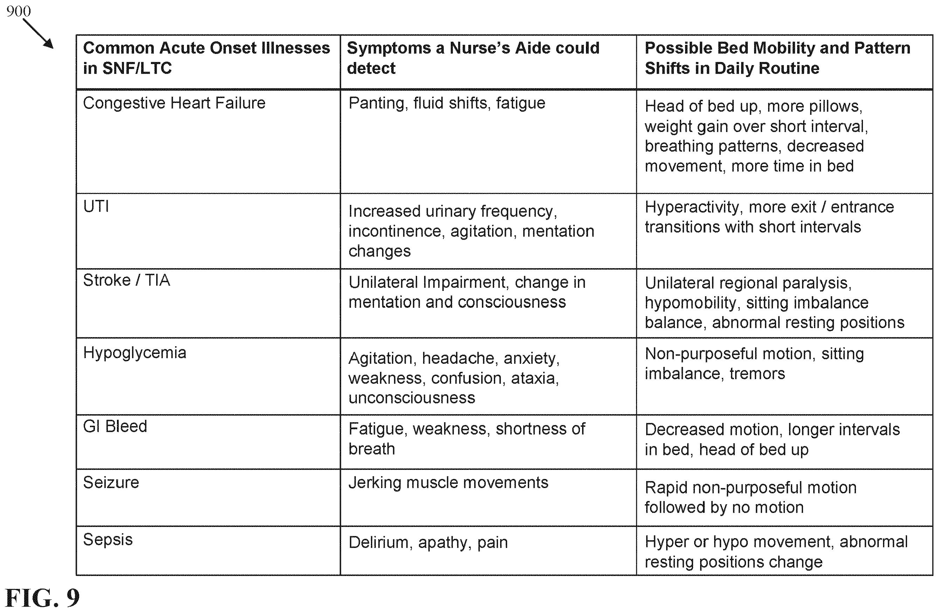

FIG. 9 illustrates a table 900 that compares the onset of acute illnesses, clinical symptoms, and potential shifts bed mobility patterns for a patient in an environment such as a long term care facility, skilled nursing facility, and the like. As an illustrative example, a patient who experiences the onset of congestive heart failure can have clinical symptoms such as panting, fluid shifts, fatigue, weight gain over a short period of time which can lead to changes in their bed mobility and daily routine such as changing the positioning the bed, decreased movement, more time spent in bed, and the like.

As another illustrative example, a patient who experiences the onset of a urinary tract infection (UTI) can have clinical symptoms such as increased urinary frequency, incontinence, agitation, changes in mental state, and the like which can lead to changes in their bed mobility and daily routine such as increased movement, increased number of bed exits, shorter time intervals between bed exits, and the like.

As another illustrative example, a patient who experiences the onset of a stroke can have clinical symptoms such as unilateral impairment, change in mental state and consciousness, and the like which can lead to changes in their bed mobility and daily routine such as unilateral regional paralysis, decreased mobility (e.g., hypomobility), sitting imbalances while on the bed, abnormal resting positions, and the like.

As another illustrative example, a patient who experiences the onset of hypoglycemia can have clinical symptoms such as agitation, headache, anxiety, weakness, confusion, ataxia, and unconsciousness which can lead to changes in their bed mobility and daily routine such as increased movement, sitting imbalances, tremors, and the like.

As another illustrative example, a patient who experiences the onset of gastrointestinal bleeding can exhibit clinical symptoms such as fatigue, weakness, shortness of breath, and the like which can lead to changes in bed mobility and daily routine such as decreased movement, longer intervals in bed, changes in positioning of the bed, and the like.

As another illustrative example, a patient who experiences the onset of a seizure can exhibit clinical symptoms such as jerking muscle movements which can lead to changes in bed mobility such as rapid non-purposeful motion followed by no motion.

As another illustrative example, a patient in a long term care facility who experiences the onset of sepsis can exhibit clinical symptoms such as delirium, apathy, pain, and the like which can lead to changes in their bed mobility and daily routine such as increased or decreased movement, abnormal resting positions, and the like.

Referring now back to FIG. 1, the system 100 can be adapted to detect changes in daily routines and patterns of daily living for the patient 102. In this embodiment, the first location 104 can be a long term care facility, skilled nursing facility, long-term acute care hospital (LTACH), or home care environment, while the second location 106 can be an acute care setting such as a hospital, or can be a medical doctor's office.

The one or more devices 110 in the system 100 can be configured as motion sensors that obtain the patient movement data 114 of the patient 102 over an extended period of time. As an illustrative example, the one or more devices 110 can include one or more types of movement tracking devices including GPS tracking devices, one or more accelerometers, or other motion-detecting technologies. As a further illustrative example, the one or more devices 110 can include bed motion sensors such as load cells or a pressure-sensing mat that is placed on a mattress of the bed that detects bed egress and ingress, duration of time spent in bed, the positioning of the bed (such as the angle of the head of the bed), and the like.

The application 118 stored in the memory of the electronic device 116 and connected to the cloud server 122 tracks the patient movement data 114 of the patient 102 and use the data to generate a daily mobility pattern as a personalized baseline 108 for the patient 102. Algorithms performed by the application 118 on the electronic device 116 or by the cloud server 122 can generate 7 day, 14 day, and 30 day trends in the daily mobility patterns of the patient 102, and these trends can be displayed for comparison with the mobility of the patient 102 during a 24 hour period to determine a likelihood of patient deterioration.

The algorithms performed by the application 118 on the electronic device 116 or by the cloud server 122 can track bed egress at mealtimes, or change in the angle of the head of the bed at mealtimes. When the patient 102 does not exit the bed and does not adjust the vertical position of the bed, the patient 102 is not likely to be eating or drinking. Decreased appetite is a marker of deterioration and can be used to generate an alarm for a caregiver to check on the patient 102, and confirm whether the patient 102 is experiencing deterioration.

The algorithms performed by the application 118 on the electronic device 116 or by the cloud server 122 can further track the total time the patient 102 has spent in bed versus the total time the patient 102 has spent out of bed, and the number of transitions into and out of bed. More time spent in bed can indicate that the patient 102 is likely sick or weak which is another marker of deterioration that can be used to generate an alarm for a caregiver.

The algorithms performed by the application 118 on the electronic device 116 or by the cloud server 122 can further track bed egress in the night hours for bathroom needs. Increased bed egress can indicate illness such as a urinary tract infection.

The algorithms performed by the application 118 on the electronic device 116 or by the cloud server 122 can further track total motion of the patient 102 while in bed. An increased amount of motion such as tossing and turning in bed can indicate agitation, hyperactivity, or insomnia, and a decreased amount of motion can indicate lethargy which are signs of patient deterioration that can be used to generate an alarm for a caregiver.

The algorithms performed by the application 118 on the electronic device 116 or by the cloud server 122 can further track daily pattern shifts such as morning waking up times including waking up earlier or later than usual, or taking naps more frequently during the day, or not positioning the head of the bed upright which are potential signs of deterioration that can be used to generate an alarm for a caregiver to check on the patient 102, and confirm whether the patient 102 is experiencing deterioration.

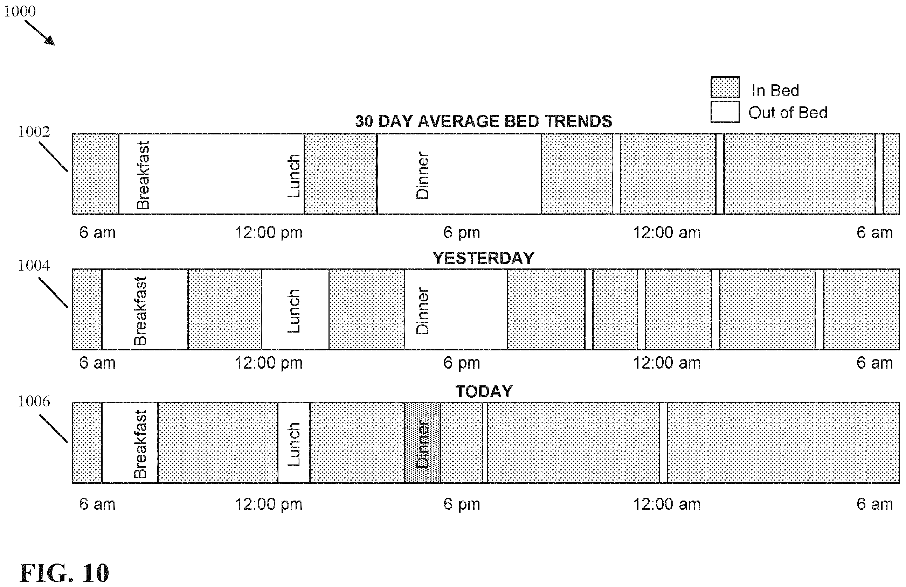

FIG. 10 illustrates a chart 1000 comparing a 30 day trend of daily mobility patterns of a patient with the mobility of the patient during various 24 hour periods of time. The chart 1000 can be displayed in a user interface on the electronic device 116 in the first location 104. The user interface on the electronic device 116 that displays the chart 1000 can be similar to the user interfaces 200, 400, 500, and 600 described above.

Additionally, the chart 1000 can also be displayed in a user interface on the terminals 126 in the second location 106. The user interface on the terminals 126 that displays the chart 1000 can be similar to the user interfaces 200, 400, 500, and 600 described above.

The chart 1000 has a first portion 1002 to display a 30 day trend of the daily mobility pattern of the patient, a second portion 1004 that displays the mobility of the patient from a previous day, and a third portion 1006 that displays the mobility of the patient from a current day. The daily mobility pattern in the first portion 1002 is a personalized baseline for the patient, and the data displayed in the second and third portions 1004, 1006 of the chart 1000 are daily mobility metrics that can be compared to the daily mobility pattern in the first portion 1002 to determine a likelihood of deterioration for the patient.

It is contemplated that the configuration of the chart 1000 may vary such that the chart 1000 can include various trends (i.e., 7 day, 14 day, 30 day, 60 day, etc.) in the first portion 1002. Also, the chart 1000 may include the personalized baseline in the first portion 1002, and include only one daily mobility metric (i.e., a daily mobility metric from yesterday or today, but not both) or may include more than two daily mobility metrics such as additional 24 hour periods of time where the movement of the patient is tracked for comparison with the personalized baseline in the first portion 1002.

In this illustrative example, first portion 1002 illustrates that the patient 102 is on average out of bed after 6am to have breakfast and lunch, returns to bed after 12pm, and then gets out of bed in the afternoon to have dinner. The patient then returns to bed in the evening for sleeping, and exits the bed for short intervals during the night hours for bathroom needs.

In this example, the second portion 1004 illustrates that during the previous day, in comparison to the daily mobility pattern in the first portion 1002, the patient left bed earlier in the morning for breakfast, then returned to bed before lunch, and then exited the bed again to have lunch. After lunch, the patient returned back to bed before leaving again to have dinner. The patient returned to bed earlier in the evening to sleep, and had more bed exists during the night hours for bathroom needs. Overall, the patient spent more time in bed during the previous day than in the personalized baseline displayed in the first portion 1002.

In this illustrative example, the third portion 1006 illustrates that in the current day the patient has spent even more time in bed compared to the previous day and the personalized baseline. For example, the patient left bed after 6am to have breakfast, returned to bed, and briefly left bed for lunch. In this example, the patient had dinner while in bed. By showing the first portion 1002, second portion 1004, and third portion 1006 together in the chart 1000, a caregiver can graphically visualize changes in the daily mobility pattern of the patient, and in particular, a significant increase in the amount of time that the patient has spent in bed. As described above, more time spent in bed can indicate that the patient is likely sick or weak which is another marker of patient deterioration.

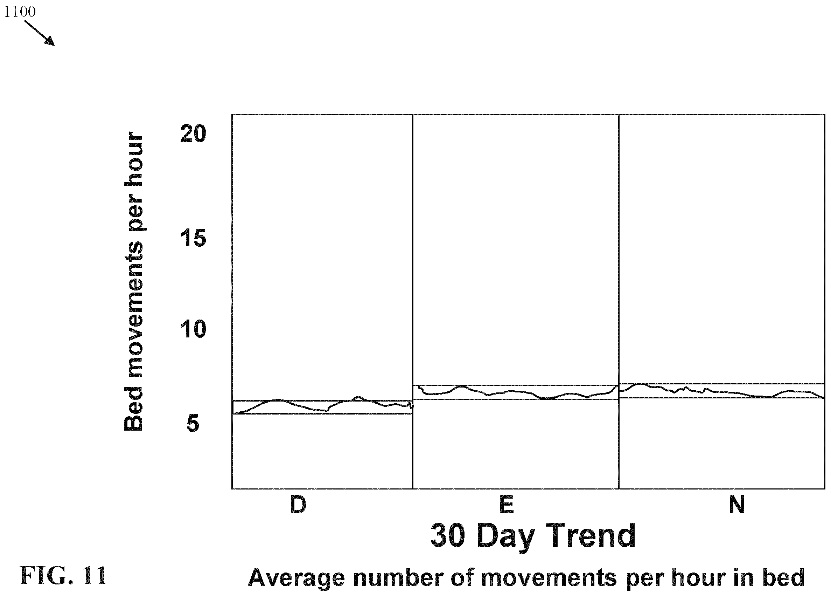

FIG. 11 illustrates a chart 1100 that displays a 30 day average number of movements per hour in bed during the day, evening, and night. In some embodiments, the 30 day trend displayed in chart 1100 is a personalized baseline for a patient used to determine a likelihood of patient deterioration. As described above, an increased amount of movement such as tossing and turning in bed can indicate agitation, hyperactivity, or insomnia, and a decreased amount of movement can indicate lethargy which are signs of patient deterioration.

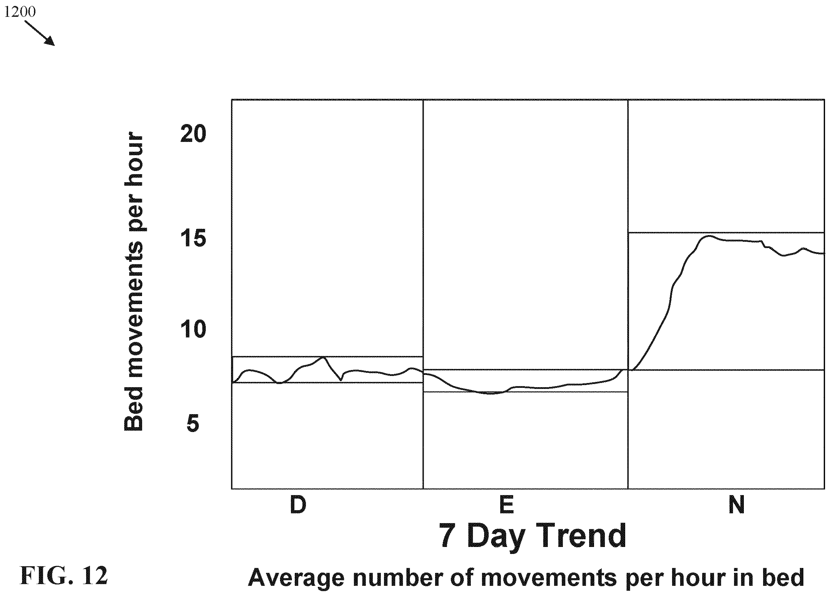

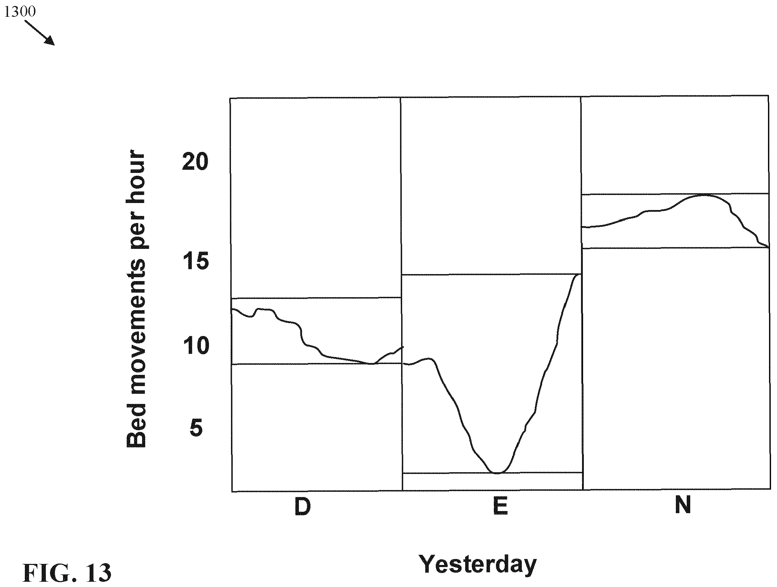

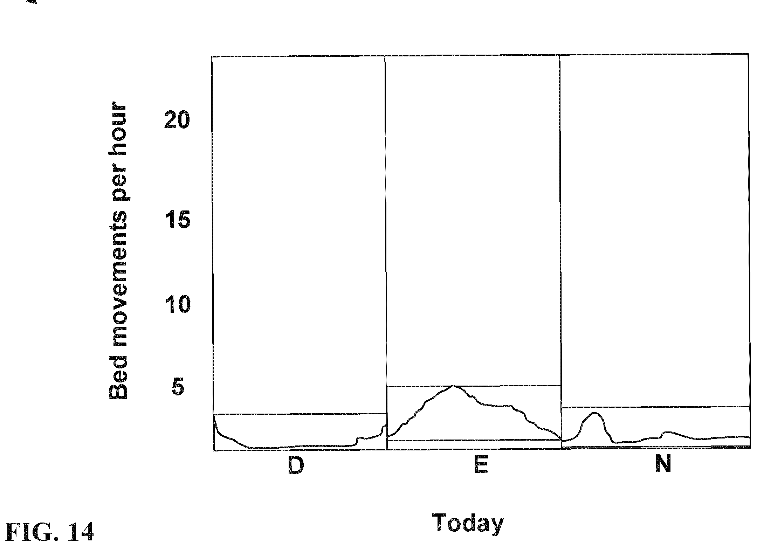

FIGS. 12, 13, and 14 illustrate charts 1200, 1300, and 1400 that display a 7 day average number of movements per hour in bed, an average number of movements per hour in bed from a previous day, and an average number of movements per hour in bed from a current day. The charts 1100, 1200, 1300, and 1400 can be displayed together in a user interface on a display screen of the electronic device 116 in the first location 104, and/or can also be displayed together in a user interface on a display screen of the terminals 126 in the second location 106. The user interfaces on the electronic device 116 and terminals 126 that display the charts 1100, 1200, 1300, and 1400 can be similar to the user interfaces 200, 400, 500, and 600 described above with regard to FIGS. 2 and 4-6, respectively.

In this illustrative example, a comparison of the chart 1200 with the chart 1100 shows that the number of movements per hour in bed for the patient has increased during the 7 day trend, especially during the night. Similarly, a comparison of the chart 1300 with the charts 1100 and 1200 shows that the number of movements per hour in bed has continued to increase during the previous day. A comparison of the chart 1400 with the charts 1100, 1200, and 1300 shows that the number of movements per hour in bed has drastically decreased during the current day. As discussed above, an increase or decrease in average movement in bed over time during the day, evening, and night can indicate patient deterioration.

In view of the foregoing, the system 100 can be used to detect changes in daily routines and patterns of daily living for a patient by establishing a personal baseline for the patient and comparing bed mobility parameters of the patent (i.e., bed egress/ingress, total time spent in bed, number of movements while in bed, etc.) to the personal baseline. The comparison between the personal baseline and the bed mobility parameters can be used to determine an early prediction of acute patient deterioration, and based on the prediction, the system 100 can generate an alarm to alert a caregiver of the need for the caregiver to check on the patient and confirm whether the patient is deteriorating. Advantageously, the system 100 can reduce stress on environments such as long term care facilities, skilled nursing facilities, long-term acute care hospitals (LTACH), and home care environments where clinical assessments by caregivers are less frequent and thorough.

Additionally, the system 100 can improve care and lower costs for high-cost, high-need populations such as nursing home residents, accelerate movement of patients to home and community based services (HCBS) over institutional care, achieve person-centered care, integrate services through care coordination and management, increase access to primary and preventive care, reduce unnecessary hospital admissions and readmissions, reduce emergency department use, and slow down the loss of function for vulnerable populations.

The use of the terms "a" and "an" and "the" and similar referents in the context of describing the subject matter (particularly in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. Furthermore, the foregoing description is for the purpose of illustration only, and not for the purpose of limitation, as the scope of protection sought is defined by the claims as set forth hereinafter together with any equivalents thereof entitled to.

The use of any and all examples, or exemplary language (e.g., "such as") provided herein, is intended merely to better illustrate the subject matter and does not pose a limitation on the scope of the subject matter unless otherwise claimed. The use of the term "based on" and other like phrases indicating a condition for bringing about a result, both in the claims and in the written description, is not intended to foreclose any other conditions that bring about that result.

Embodiments of the invention can be described with reference to the following numbered clauses, with preferred features laid out in the dependent clauses:
Clause 1. A method of comparing a measured physiological parameter to a personalized baseline of a patient, the method comprising:
   aggregating physiological parameter data over a period of time at one or more first locations;
   determining a personalized baseline based on the aggregated physiological parameter data; and
   generating a user interface that displays a comparison of a physiological parameter measured at a second location to the personalized baseline.
Clause 2. The method of Clause 1, further comprising displaying a visualization curve comparing the physiological parameter measured at the second location to the personalized baseline in a window of the user interface.
Clause 3. The method of Clause 1 and 2, wherein the physiological parameter data includes patient movement data, and the method further comprises:
   using the patient movement data to generate a daily mobility pattern as a personalized baseline for the patient.
Clause 4. The method of Clause 3, further comprising comparing mobility of the patient during a 24 hour period to the daily mobility pattern to determine a likelihood of patient deterioration.
Clause 5. The method of Clause 4, further comprising displaying the comparison of the mobility of the patient during the 24 hour period to the daily mobility pattern in the user interface, the user interface being generated on a device in the first or second location.
Clause 6. The method of any one of the preceding Clauses, further comprising determining personalized baselines for physiological parameters including heart rate, respiration rate, blood pressure, temperature, blood oxygen saturation (SpO2), end-tidal CO2 (EtCO2), electrocardiogram (ECG) data, blood glucose level, partial thromboplastin time (PTT), prothrombin time (INR), hemoglobin level (A1c), patient weight, and patient movement.
Clause 7. The method of any one of the preceding Clauses, further comprising assigning confidence intervals to the physiological parameter data depending on the type of device used to measure the physiological parameter data.
Clause 8. The method of Clause 7 when dependent on Claim 2, wherein displaying a visualization curve includes displaying the personalized baseline as boundaries representing the confidence intervals.
Clause 9. The method of any one of the preceding Clauses, further comprising customizing the personalized baselines based on a status of a patient including when the patient is at rest, when the patient is active, after the patient concludes an activity, and when the patient is sleeping.
Clause 10. The method of any one of the preceding Clauses, further comprising customizing the personalized baselines based on previous hospitalization discharge data. Clause 11. The method of any one of the preceding Clauses, wherein the first location is different from the second location, and the first and second locations are a hospital, a long-term-care facility, a skilled nursing facility, a physician's office, or the patient's home.
Clause 12. The method of any one of the preceding Clauses, wherein aggregating physiological parameter data comprises collecting at least one physiological parameter continuously or periodically.
Clause 13. The method of any one of the preceding Clauses, wherein the personalized baseline is determined on a server.
Clause 14. The method of any one of the preceding Clauses, wherein the personalized baseline is at least one of:
   an average value;
   a range of values;
   a median value; or
   a most frequently repeated value, of the aggregate physiological parameters.

## Claims

1. A system for determining personalized baselines for use across multiple locations and by multiple caregivers, the system comprising:
at least one processor; and
memory encoding instructions which, when executed by the at least one processor, cause the at least one processor to:
aggregate physiological parameters measured at a first location;
determine a personalized baseline based on the aggregated physiological parameters; and
generate a user interface that compares physiological parameters measured at a second location to the personalized baseline.

2. The system of claim 1, further comprising at least one sensor configured to collect at least one of the physiological parameters continuously or periodically.

3. The system of claim 1 or 2, further comprising a server on which the personalized baselines are determined.

4. The system of claim 1, 2, or 3, wherein the personalized baseline is at least one of:
an average value;
a range of values;
a median value; or
a most frequently repeated value, of the aggregated physiological parameters.

5. The system of any of the preceding claims, wherein the first location is different from the second location, and the first and second locations are selected from a group consisting of a hospital, long-term-care facility, skilled nursing facility, physician office, and a patient's home.

6. The system of any of the preceding claims, wherein the personalized baselines are customizable based on a status of a patient including when the patient is at rest, when the patient is active, after the patient concludes an activity, and when the patient is sleeping.

7. The system of any of the preceding claims, wherein the personalized baselines are customizable based on previous hospitalization discharge data.

8. The system of any of the preceding claims, wherein the system analyzes changes to the personalized baselines by one or more types of interventions.

9. The system of any of the preceding claims, wherein the memory encodes additional instructions which, when executed by the at least one processor, cause the at least one processor to:
assign confidence intervals to the physiological parameters measured at the first location, the confidence intervals assigned based on the type of device used to measure the physiological parameters.

10. The system of any of the preceding claims, wherein the user interface includes one or more windows that display visualization curves that compare the physiological parameters measured at the second location to the personalized baselines.

11. The system of claim 10 when dependent on claim 9, wherein the personalized baselines are displayed in the one or more windows as boundaries representing the confidence intervals.

12. The system of any of the preceding claims, further comprising bed motion sensors to detect patient movement data including bed egress and ingress, duration of time spent in bed, and the positioning of the bed;
wherein the physiological parameters include the patient movement data; and
wherein the patient movement data is used to generate a daily mobility pattern as a personalized baseline for the patient.

13. The system of claim 12, wherein mobility of the patient during a 24 hour period is compared to the daily mobility pattern to determine a likelihood of patient deterioration, and
preferably wherein the comparison of the mobility of the patient during the 24 hour period to the daily mobility pattern is displayed in the user interface, and the user interface is generated on a device in the first or second location.

14. The system of any of the preceding claims, wherein the personalized baselines for physiological parameters including heart rate, respiration rate, blood pressure, temperature, blood oxygen saturation (SpO2), end-tidal CO2 (EtCO2), electrocardiogram (ECG) data, blood glucose level, partial thromboplastin time (PTT), prothrombin time (INR), hemoglobin level (A1c), patient weight, and patient movement.

15. A method of comparing a measured physiological parameter to a personalized baseline of a patient, the method comprising:
aggregating physiological parameter data over a period of time at one or more first locations;
determining a personalized baseline based on the aggregated physiological parameter data; and
generating a user interface that displays a comparison of a physiological parameter measured at a second location to the personalized baseline.
